# EUROPEAN PATENT APPLICATION

(11) **EP 0 770 393 A2**
(43) Date of publication of application: **02.05.1997**
(21) Application number: 96307537.9
(22) Date of filing: 17.10.1996
(51) Int. Cl.: A61K 35/28, A61K 31/435

(54) **Prolongation of organ transplantation using rapamycin loaded donor splenocytes**

(30) Priority: 27.10.1995 US 5932
(71) Applicant: AMERICAN HOME PRODUCTS CORPORATION, Madison, New Jersey 07940-0874 (US)
(72) Inventor: Armstrong, Jay Joseph, Bensalem, Pennsylvania 19020 (US); Merrill, Diana Susan, Warren, New Jersey 07059 (US)
(74) Representative: Wileman, David Francis, Dr.

(57) **Abstract**

The invention disclosed herein provides a method of prolonging organ or tissue transplantation survival times in mammals via infusion of an effective amount of rapamycin-loaded donor splenocytes to the organ or tissue recipient.

## Description

### Field of Invention

This invention provides a specifically defined method for the prolongation of organ or tissue transplant survival by precisely targeting the donor-reactive host lymphocyte population. The effect is accomplished through the administration of rapamycin-loaded donor splenocytes to the organ or tissue recipient.

### Background of the Invention

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Seghal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Rapamycin has been shown to be effective in inhibiting transplant rejection (U.S. Patent Application Ser. No. 362,544 filed June 6, 1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); and R. Y. Calne et al., Lancet 1183 (1978)]. A method for inducing immunosuppression by administration of rapamycin-loaded red blood cells is disclosed in our commonly owned U. S. patent application no. 07/998,065, filed December 22, 1992.

The evaluation of immunosupppressive agents has contributed greatly to our understanding of the significance of T-cell activity in graft survival. The effects of corticosteroids and cyclosporin on the immune system is discussed in J. F. Bach and T. B. Strom, *The Mode of Action of Immunosuppressive Agents,* New York: Elsevier Press, 1985, pages 72-78 and 250-254 respectively. Hess *et al., Journal of Immunology 128* (1), 355-367 (1982) discuss the effect of Cyclosporin A on human lymphocyte responses in vitro. The clinical usefulness of antilymphocyte antibodies is discussed by A. B. Cosimi, *Transplantation* *Proceedings,* XV (1), 583-589 (1983). Babany *et al., J. Pharm. Exp. Ther.* 244 (1), 259-262 (1988) discusses the *in vivo* use of cyclosporine in the mouse heart transplant model.

### Summary of the Invention

According to this invention, splenocytes (lymphocytes isolated from the spleen) of donor mammals, are incubated with rapamycin and administered to the organ or tissue recipient mammal to provide immunosuppression and prevent or treat graft rejection. In the allograft procedures disclosed herein, neonate hearts from Brown Norway rats are transplanted into ear pockets in Lewis rats and the Lewis rats are administered splenocytes obtained from the Brown Norway rats which have been loaded with rapamycin according to the method of this invention on days 4 and 13 post-transplant. In addition to the immunosuppression afforded by rapamycin-loaded splenocytes in allograft procedures, it is believed that the immunosuppression procedure disclosed herein will be applicable to xenograft transplant procedures.

### Detailed Description of the Invention

### 1. Cardiac engraftment in recipient male Lewis rat

The following detailed procedures are included to illustrate the method of this invention. It is envisioned that other mammals and tissues or organs therefrom, including humans, can be used in the method of this invention. The various solutions, surgical procedures and laboratory procedures are well known to those skilled in the art.

Male Lewis rat recipients (Charles River), 325 g., received an intraperitoneal (i.p.) injection of 20 milligrams of Pentobarbital (Nembutal, Abbott Laboratories) prior to neonatal heart implantation. The left ear was swabbed with Betadine (povidine iodine) using sterile cotton applicators. One milliliter of sterile RPMI 1640 culture media (Gibco) was injected into the ear between the ventral cartilage plate and the epidermis, producing a raised bleb. Using a sterile number 11 scalpel, a stab incision was made along the posterior margin and parallel to the plane of the cartilage plate. Any remaining media was gently expressed from the pocket using a sterile cotton applicator. Mixed sex neonate Brown Norway (BN) rats (Charles River) two to five days old were employed as cardiac donors. The thorax and cervical regions were swabbed with 70% 2-propanol and Betadine and donors were sacrificed by decapitation. Two penetrating incisions were made along the lateral margins of the rib cage which was then reflected caudally, exposing the thoracic cavity. The heart was dissected free of all vascular and thymic connections and was placed in sterile iced RPMI 1640. At this point the neonatal heart was evaluated for any gross malformations or reduced activity. The heart was divided along the midline from the apex to the root of the aorta, producing two approximately equal sections. A single section consisting of atrial and ventricular tissue was used for each recipient rat and once inserted into the ear pocket, the flap was gently closed and sealed witha drop of flexible colloidion (Sigma) using a sterile cotton applicator.

### 2. Preparation of rapamycin-loaded Brown Norway splenocytes

Three male 350 gram BN rats were terminally anesthetized with excess nembutal i.p. Spleens were then removed under sterile conditions, and placed into RPMI 1640 media (Gibco) at room temperature. Each spleen was then placed into a 100 mm sterile petri dish containing 10 mls of sterile RPMI 1640 and macerated through a mesh screen. The petri dish was then washed with 5 ml of sterile RPMI media after all spleens were disrupted and the wash was added to a 50 ml centrifuge tube. The crude preparation was then centrifuged at 800 rpm (100xG) in a Sorvall RT 6000 centrifuge for 10 minutes at room temperature. The supernatant was withdrawn and the pellet resuspended in 20 mls of fresh RPMI 1640 and again centrifuged as under the previous conditions. The supernatant was again drawn off and the pellet resuspended in 15 mls of Tris-ammonium chloride lysis buffer at room temperature. After four minutes, 30 mls of RPMI 1640 was added and the preparation was centrifuged at 800 rpm (100xG) for 10 minutes at room temperature. The supernatant was drawn off and the pellet resuspended in 40 mls of fresh RPMI and centrifuged as before. The supernatant was again drawn off and the pellet resuspended in 2 mls of fresh media. This 2 ml volume was then carefully layered over 10 mls of Lympholyte-R (Cedarlane Labs) at room temperature in a centrifuge tube and centrifuged at 2,000 (800xG) for 20 minutes. The splenocytes were extracted from the Lympholyte-media interface by gentle aspiration and added to 30 mls of fresh RPMI media at room temperature. This suspension was washed twice at 800 rpm (100xG) for 10 minutes. The second supernatant wash was discarded and the cells were resuspended in 5 mls of fresh RPMI media for counting. The media volume was adjusted to produce an incubation mixture of 125 x 10⁶ splenocytes/ml. Rapamycin was incubated at 75 °C in absolute ethyl alcohol (10 mg/100 µl) until completely dissolved. The entire volume of this mixture was then slowly added to 4.9 mls of splenocyte suspension, gently mixed, and incubated at 37 °C for 30 minutes. The incubation mixture was then centrifuged at 800 rpm (100xG) for 10 minutes at room temperature, the supernatant discarded, and the pellet washed for five separate cycles at 800 rpm with 30 ml portions of fresh RPMI to remove unbound rapamycin. Transplanted rats received 0.200 ml of loaded donor splenocytes (equivalent to 5x10⁶ cells) via a tail vein infusion on post-operative days 4 and 13 only.

### 3. Determination of cardiac allograft survival

Graft survival monitoring of transplanted cardiac tissue contractility was accomplished using the Gould RS3200 chart recorder and was begun on post-operative day five and continued on alternating days until point of failure. Control animals were evaluated in an identical format but received only non-rapamycin-treated donor splenocyte transfusion on days one, four and seven. Graft failure is generally defined as a flat line chart response obtained from multiple bipolar recording sites, sustainable over a 48 hour period. Animals in any treatment group exhibiting graft failure prior to six days (the mean failure point for the control group) were excluded from consideration in the study and were considered technical failures.

### 4. Determination of raparnycin concentration in splenocytes

Two separate samples of rapamycin loaded splenocytes were provided for high pressure liquid chromatography (HPLC) analysis on a Supelco LC-18 column (4.6 x 250 mm). The analyses showed that the average concentration of rapamycin was 178 µg/ml of the loaded splenocyte samples. Through extension, this implies that a concentration of approximately 35.6 µg/day/animal or 101.7 µg/kg/day was transfused on days 4 and 13.

### 5. Conclusions

Tail vein administration of 0.200 ml of splenocytes containing 35.6 µg rapamycin for two days produced a 17 day mean survival time (16.5 day median) for neonatal heart allografts in male Lewis recipients. By comparison, the mean survival time for the control animals was 6.3 days. Mantel-Haenszel survival analysis produced a highly significant difference in survival times between the two groups (p < 0.001). The mean survival time for animals received an oral dosing of rapamycin in Phosal for 14 days at a dose of 900 µg/kg was 15.6 days. Transplant animals received rapamycin loaded red blood cells for 7 days at 180 µg/kg had a mean survival time of 28.1 days. It is concluded that the method of this invention has substantial clinical application in the pre-transplant or immediate post-transplant phase of treatment in organ transplantation.

### Pharmacology

Based on the data reported herein, it is anticipated that a daily dose of from 0.01 to 10 mg/kg/day of rapamycin in rapamycin-loaded splenocytes, given as many days as necessary, will substantially prolong transplantation survival rates. The exact dosage will depend on , among other factors, the age and condition of the recipient animal and degree of efficacy of rapamycin in that animal, and will, or course, be determined by qualified medical personnel.

### Pharmaceutical Composition

It is anticipated that rapamycin-loaded splenocytes can be administered in various liquid media which are physiologically compatible with the mammal being treated and includes suspensions of rapamycin-loaded splenocytes in donor blood, blood plasma or blood serum obtained from the organ donor or a cross-matched third party donor, biological media compatible with blood such as RPMI 1640, normal saline or 5% dextrose in water, along with buffers, electrolytes, viscosity-adjusting additives, antibiotics, and other immunosuppressing agents amenable to intravenous administration.

## Claims

1. A pharmaceutical composition for suppressing the immune system in a mammal receiving an organ or tissue transplant which comprises rapamycin-loaded splenocytes suspended in a physiologically acceptable liquid carrier.

2. A pharmaceutical composition as claimed in Claim 1 wherein the rapamycin-loaded splenocytes are obtained from the donor.

3. A composition according to Claims 1 or Claim 2 in unit dosage form containing a therapeutically effective amount of rapamycin-loaded splenocytes.

4. Extracorporeal splenocytes loaded with rapamycin.

5. Splenocytes according to Claim 4 which are substantially saturated with rapamycin.

6. A process for preparing rapamycin-loaded splenocytes which comprises isolation of splenocytes from donor spleen, incubation of the splenocytes with a solution of rapamycin and suspending rapamycin-loaded splenocytes in a physiologically compatible liquid carrier for intravenous administration to the organ or tissue recipient.

7. A process for preparing rapamycin-loaded splenocytes as claimed in Claim 6 wherein the splenocytes are washed to remove unbound rapamycin before they are suspended in the physiologically compatible liquid carrier.

8. Use of rapamycin-loaded splenocytes to prepare a composition for prolonging organ or tissue graft survival time in a mammal.

9. Use of rapamycin-loaded splenocytes to prepare a medicament for immunosuppression in a mammal.
